(19) Europäisches Patentamt
European Patent Office
Office européen des brevets

(11) Veröffentlichungsnummer : **0 374 416 B1**

(12) # EUROPÄISCHE PATENTSCHRIFT

(45) Veröffentlichungstag der Patentschrift : **07.01.93 Patentblatt 93/01**

(51) Int. Cl.$^5$ : **C07C 49/603**

(21) Anmeldenummer : **89119450.8**

(22) Anmeldetag : **20.10.89**

(54) **Verfahren zur Herstellung von 2,6,6-Trimethyl-cyclohex-2-en-1,4-dion.**

(30) Priorität : **17.12.88 DE 3842547**

(43) Veröffentlichungstag der Anmeldung : **27.06.90 Patentblatt 90/26**

(45) Bekanntmachung des Hinweises auf die Patenterteilung : **07.01.93 Patentblatt 93/01**

(84) Benannte Vertragsstaaten : **AT BE CH DE FR GB IT LI NL**

(56) Entgegenhaltungen :
**EP-A- 0 311 408**
**DE-A- 2 515 304**
**DE-A- 2 657 386**
**DE-B- 2 457 157**
**DE-B- 2 526 851**
**FR-A- 2 335 486**

(73) Patentinhaber : **HÜLS AKTIENGESELLSCHAFT**
**Patentabteilung / PB 15 - Postfach 13 20**
**W-4370 Marl 1 (DE)**

(72) Erfinder : **Bellut, Hans, Dr.**
**Bergstrasse 50**
**W-4408 Dülmen (DE)**

**EP 0 374 416 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

2,6,6-Trimethyl-cyclohex-2-en-1,4-dion (Keto-isophoron, KIP) ist eine wertvolle Ausgangssubstanz für Synthesen der Terpen-Chemie. Es kann sowohl als bequeme und wohlfeile Basis zur Herstellung von Vitaminkörpern der A-Reihe und von Vitamin-E dienen, als auch zur Gewinnung naturidentischer Duft- und Aromastoffe (vgl. z. B. O. Isler, Carotenoids, Birkhäuser Verlag Basel und Stuttgart, 1971). Die Synthese von KIP erfolgt zweckmäßig durch katalytische Sauerstoff- Oxydation von 3,5,5-Trimethyl-cyclohex-2-en-1-on (alpha-Isophoron) bzw. besser von 3,5,5-Trimethyl-cyclohex-3-en-1-on (beta-Isophoron), siehe z. B. DE-A 25 26 851, NL-OS 74 15 848 und DE-OS 24 57 157, DE-OS 25 15 304. Im ersten Falle entstehen jedoch zahlreiche Nebenprodukte, die die Aufarbeitung erschweren, im zweiten Falle war die Basis der Gewinnung von beta-Isophoron bislang nicht zufriedenstellend gelöst, außerdem ist die Herstellung zu aufwendig, falls gute Ergebnisse erzielt werden sollen, im Hinblick auf Menge und Qualität des zu erhaltenden KIP.

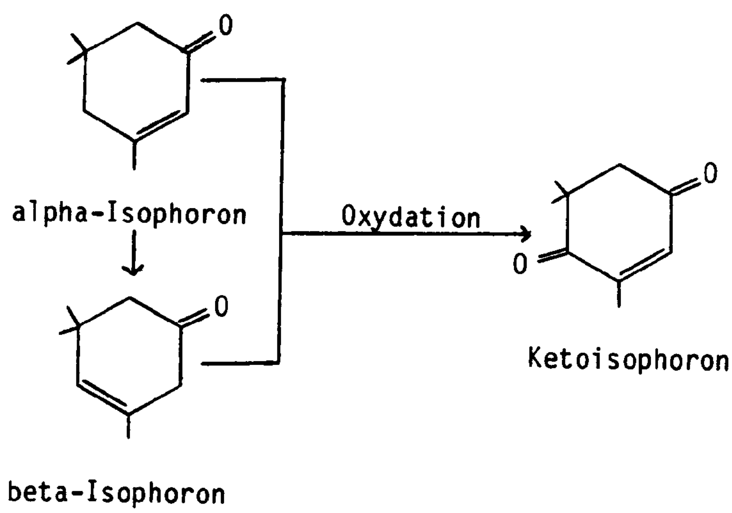


Der Engpaß zur Gewinnung von beta-Isophoron wurde durch ein neues Verfahren zur Isomerisierung von alpha-Isophoron gemäß DE-OS 37 35 211 beseitigt, so daß nun Ausgangsprodukt in genügender Menge und preiswert zur Verfügung steht.

Die Oxydation von beta-Isophoron zu KIP wird gemäß DE-OS 25 15 304 mit Sauerstoff oder einem sauerstoffhaltigen Gas mit Hilfe eines Blei-, Vanadium-, Chrom-, Mangan-, Eisen- oder Kobaltsalzes, z.B. eines Acetylacetonates, gegebenenfalls in Gegenwart einer organischen Stickstoffbase, z. B. Pyridin, durchgeführt.

Die gängigen Übergangsmetalle sind jedoch für die vorliegende Reaktion weniger geeignet, da sie gemäß DE-OS 37 35 211 die Einstellung des Gleichgewichts alpha-Isophoron/beta-Isophoron besonders gut katalysieren und damit zur erheblichen Rückisomerisierung führen.

Im Falle der DE-OS 25 15 304 wird mit großen Überschüssen an Stickstoffbasen gearbeitet - Molverhältnis beta-Isophoron/Pyridin 130/330 - was zu einer Ausbeuteerhöhung und Unterdrückung von Nebenprodukten führen soll. Zudem wird noch die Zugabe eines weiteren Lösungsmittels empfohlen. Die Entfernung der großen Mengen an Stickstoffbasen ist mit einem größeren destillativen Aufwand verbunden.

Weiterhin ist es gemäß DE-OS 24 57 157 bekannt, bei der Luftoxydation von beta-Isophoron zu KIP Cu(II)-Acetylacetonat als Katalysator zu verwenden, jedoch werden lediglich Ausbeuten von nur ca. 50 % erzielt.

Es war daher Aufgabe der Erfindung, ein Verfahren zur Herstellung von KIP mittels geeigneter Katalysatoren aus beta-Isophoron zu finden, das zu guten Ausbeuten führt, bei dem keine zusätzlichen Lösungsmittel verwendet werden müssen und - wenn möglich - die Menge an zuzusetzender Stickstoffbase reduziert werden kann.

Gegenstand der Erfindung ist daher ein Verfahren zur Herstellung von 2,6,6-Trimethyl-cyclohex-2-en-1,4-dion durch katalytische Oxydation von beta-Isophoron mit Sauerstoff oder Sauerstoff enthaltenden Gasen mittels eines Metallkatalysators und einer Stickstoffbase, welches dadurch gekennzeichnet ist, daß als Katalysator Cu(II)-Acetylacetonat unter Zusatz von Pyridin in Abwesenheit weiterer Lösemittel eingesetzt werden.

Überraschenderweise ist im erfindungsgemäßen Verfahren durch die Anwendung des Cu-Katalysators also nur eine sehr begrenzte Menge an Stickstoffbase nötig. Gegenüber dem bekannten Verfahren, bei dem ausdrücklich darauf hingewiesen wird, die Konzentration des Einsatzproduktes im Hinblick auf hohe Ausbeuten niedrig zu halten, handelt es sich erfindungsgemäß um eine Reduzierung um den Faktor 10 und damit um einen

verringerten technischen Aufwand bei der Entfernung der Base, ohne daß Ausbeuteverluste hingenommen werden müssen. Zudem entfällt der Zusatz weiterer Lösungsmittel, es kann sogar schädlich sein (Isopropanol).

Es empfiehlt sich, bei einem Molverhältnis beta-Isophoron/Pyridin/Cu-Komplex von 100-150/20-50/0,1-5, bevorzugt 125-135/25-40/0,5-1,5, zu arbeiten.
Das besonders bevorzugte Molverhältnis von beta-Isophoron/Pyridin/Cu-Komplex beträgt ca. 130/30/1.

Die praktische Versuchsführung kann auf verschiedene Art und Weise vorgenommen werden, indem entweder in das turbinierte Reaktionsgemisch bei 20 bis 100 °C, insbesondere bei 50 bis 80 °C, überschüssiger Sauerstoff bzw. Luft eingeleitet wird. Die Katalysatormenge sollte dabei 0,1 bis 5 Gew.-% Kupferkomplex - insbesondere 0,5 bis 1,5 % - bezogen auf eingesetztes beta-Isophoron, betragen. Bei einer anderen Variante pumpt man das Reaktionsgemisch über eine entsprechend dimensionierte beheizbare Säule, die mit Glaswendeln gefüllt ist, im Kreis und leitet Sauerstoff bzw. Luft im Gegenstrom durch die Anordnung. Das Fortschreiten der Reaktion kann gaschromatografisch verfolgt werden, bis das eingesetzte beta-Isophoron praktisch verbraucht ist. Auf diese Art wurden Umsätze bis zu 97 % eingesetztes beta-Isophoron erzielt. Dabei entstehen ca. 1,5 % alpha-Isophoron, 85 bis 90 % KIP und ca. 10 % nicht näher untersuchte Überkondensate. Da sowohl alpha- als auch beta-Isophoron erneut auf Einsatzprodukt aufgearbeitet werden können, ist die Ausbeute bei diesem Prozeß 90 % und mehr.

Beispiel 1

In einer Begasungsapparatur wurden 176 g 93 %iges beta-Isophoron, 22 g Pyridin und 1,76 g Cu-Acetylacetonat unter Turbinieren 1 500 U/min auf 60 °G temperiert. Dann leitete man acht Stunden Sauerstoff mit einer Geschwindigkeit von 39 1 $0_2$/h ein. Nach Anspringen der Reaktion steigt die Innentemperatur zunächst auf ca. 75 °C, um im Laufe der Zeit wieder langsam auf die Badtemperatur abzufallen. Das Fortschreiten der Reaktion wurde durch stündliche Probenahme und GC-Untersuchung verfolgt.

| Zeit h | beta-IP % (GC) | alpha-IP % (GC) | KIP % (GC) | Überkondens. % (GC) | Ausb. KIP % |
|---|---|---|---|---|---|
| 0 | 93 | 5 | - | - | - |
| 1 | 69 | 4 | 24 | 3 | 26 |
| 2 | 41 | 3 | 48 | 7 | 52 |
| 3 | 24 | 3 | 63 | 9 | 69 |
| 4 | 11 | 3 | 76 | 10 | 81 |
| 5 | 6 | 3 | 81 | 10 | 87 |
| 6 | 3 | 3 | 83 | 11 | 89 |
| 7 | 2 | 3 | 84 | 11 | 90 |
| 8 | 1 | 3 | 84 | 11 | 90 |

IP = Isophoron

Die Aufarbeitung erfolgt zunächst durch eine Vordestillation im Vakuum, um alle flüchtigen Bestandteile vom Rückstand 13,4 g, bestehend aus Überkondensaten und Katalysator, zu trennen. Daran anschließend erfolgt eine Fraktionierung.

I $Kp_3$ = 65 - 69 °C; 18,6 g mit 74 % KIP und 26 % alpha- und beta-Isophoron

II $Kp_3$ = 69 - 73 °C; 134,1 g mit 98 % KIP

III Rückstand 1,5 g

Das verwendete Pyridin findet sich als Kühlfalleninhalt bei der Vordestillation und bei der Fraktionierung.

Vergleichsbeispiele 2 bis 5

In der gleichen Art wie Beispiel 1 wurden Versuche mit verschiedenen Katalysatoren durchgeführt. Die Reaktionsmischung wurde nach beendeter Umsetzung gaschromatografisch untersucht. Die Ergebnisse sind in nachfolgender Tabelle zusammengefaßt.

| Beispiel | Katalysator* | Reaktionszeit Stdn. | Produktverteilung % beta-IP | alpha-IP | KIP | Überk. |
|---|---|---|---|---|---|---|
| 2 | FE-Acetyl-acetonat | 8 | 24,2 | 6,7 | 51,3 | 17,7 |
| 3 | $VOSO_4$ | 15 | 8,2 | 15,9 | 59,5 | 16,1 |
| 4 | VO-Acetyl-acetonat | 15 | 5,8 | 8,6 | 73,8 | 12,0 |
| 5 | Cu-Acetyl-acetonat | 15 | 10,9 | 4,4 | 60,5 | 24,2 |

* Der Katalysator wurde in jeweils 22 g Isopropylalkohol gelöst.

## Patentansprüche

1. Verfahren zur Herstellung von 2,6,6-Trimethyl-cyclohex-2-en-1,4-dion durch katalytische Oxydation von beta-Isophoron mit Sauerstoff oder Sauerstoff enthaltenden Gasen mittels eines Metallkatalysators und einer Stickstoffbase,
dadurch gekennzeichnet,
daß als Katalysator Cu(II)-Acetylacetonat unter Zusatz von Pyridin in Abwesenheit weiterer Lösemittel eingesetzt werden.

2. Verfahren nach Anspruch 1,
dadurch gekennzeichnet,
daß das Molverhältnis beta-Isophoron/Pyridin/Cu-Komplex 100-150/20-50/0, 1-5 beträgt.

3. Verfahren nach Anspruch 2,
dadurch gekennzeichent,
daß das bevorzugte Molverhältnis beta-Isophoron/Pyridin/Cu-Komplex 125-135/25-40/0,5-1,5 beträgt.

4. Verfahren nach Anspruch 2,
dadurch gekennzeichnet,
daß besonders bevorzugt das Molverhältnis beta-Isophoron/Pyridin/Cu-Komplex 130/30/1 beträgt.

## Claims

1. A process for manufacturing 2,6,6-trimethyl-cyclohex-2-ene-1,4 -dione by catalytic oxidation of beta-isophorone with oxygen or oxygen-containing gases by means of a metal catalyst and a nitrogen base, characterized in that Cu(II)-acetylacetonate is used as the catalyst in the presence of pyridine and in the absence of any other solvent.

2. A process according to claim 1, characterized in that the molar relationship beta-isophorone/pyridine/Cu-complex is 100-150/20-50/0.1-5.

3. A process according to claim 2, characterized in that the preferred molar relationship beta-isophorone/pyridine/Cu-complex is 125-135/25-40/0.5-1.5.

4. A process according to claim 2, characterized in that the particularly preferred molar relationship beta-isophorone/pyridine/Cu-complex is 130/30/1.

## Revendications

1. Procédé d'obtention de la 2,6,6-triméthyl-cyclohex-2ème-1,4-dione par oxydation catalytique de la bêta-isophoron avec de l'oxygène ou des gaz contenant de l'oxygène, à l'aide d'un catalyseur métallique et d'une base azotée, caractérisé en ce que comme catalyseur, l'acétylacétonate de cuivre (II) est mis en oeuvre, tout en ajoutant de la pyridine en l'absence de tout solvant supplémentaire.

2. Procédé selon la revendication 1, caractérisé en ce que le rapport molaire bêta-isophoron/pyridine/complexe cuivrique s'élève à 100-150/20-50/0,1-1,5.

3. Procédé selon la revendication 2, caractérisé en ce que le rapport molaire préféré bêta-isophronone/pyridine/complexe cuivrique s'élève à 125-135/25-40)0,5 - 1,5.

4. Procédé selon la revendication 2, caractérisé en ce que d'une manière particulièrement préférée, le rapport molaire bêta-isophoron/pyridine/complexe cuivrique s'élève à 130-30-1-.